# EUROPEAN PATENT SPECIFICATION

(11) **EP 1 740 164 B1**
(45) Date of publication and mention of the grant of the patent: **13.08.2008**
(21) Application number: 05738648.4
(22) Date of filing: 25.04.2005
(51) Int. Cl.: A61K 31/366, A61K 9/00, A61P 27/06

(54) **STATINS FOR THE TREATMENT OF OCULAR HYPERTENSION AND GLAUCOMA**
STATINE ZUR BEHANDLUNG VON OKULARER HYPERTONIE UND GLAUKOM
STATINES POUR LE TRAITEMENT DE L'HYPERTENSION OCULAIRE ET DU GLAUCOME

(30) Priority: 26.04.2004 US 565469 P
(43) Date of publication of application: 10.01.2007
(73) Proprietor: Alcon, Inc., 6331 Hünenberg (CH)
(72) Inventor: HELLBERG, Mark, R., Arlington, Texas 76017 (US); FLEENOR, Debra, L., Crowley, Texas 76036 (US); SHEPARD, Allan, Fort Worth, Texas 76132 (US); PANG, Iok-Hou, Grand Prairie, Texas 75052 (US)
(74) Representative: Hanna, Peter William Derek
(86) International application number: PCT/US2005/014255
(87) International publication number: WO 2005/105069

(56) References cited:
- WO-A-20/05053683
- DATABASE WPI Section Ch, Week 200437 Derwent Publications Ltd., London, GB; Class B04, AN 2004-396426 XP002342021 & JP 2004 149480 A (SAISENTAN IGAKU KENKYUSHO KK) 27 May 2004 (2004-05-27)
- MCGWIN JR G ET AL: "Statins and other cholesterol-lowering medications and the presence of glaucoma" ARCHIVES OF OPHTHALMOLOGY 2004 UNITED STATES, vol. 122, no. 6, 2004, pages 822-826, XP009052866 ISSN: 0003-9950

## Description

### BACKGROUND OF THE INVENTION

The present invention relates to glaucoma and controlling or lowering intraocular pressure in patients. More particularly, the present invention relates to the use of pharmaceutical compositions which are useful in the treatment of glaucoma and controlling or lowering intraocular pressure.

Glaucomatous optic neuropathy (glaucoma) is a disease characterized by the permanent loss of visual function due to irreversible damage to the optic nerve. The several morphologically or functionally distinct types of glaucoma are typically characterized by elevated intraocular pressure (IOP), which is considered to be causally related to the pathological course of the disease. Ocular hypertension is a condition wherein IOP is elevated, but no apparent loss of visual function has occurred; such patients are considered to be at high risk for the eventual development of the vision loss associated with glaucoma. Some patients with glaucomatous field loss have relatively low IOP. These normal tension or low tension glaucoma patients can also benefit from agents that lower and control IOP. If glaucoma or ocular hypertension is detected early and treated promptly with medications that effectively reduce elevated intraocular pressure, the loss of visual function or its progressive deterioration can generally be ameliorated. Drug therapies that have proven to be effective for the reduction of intraocular pressure include both agents that decrease aqueous humor production and agents that increase the outflow facility.

Continuously elevated IOP has been associated with the progressive deterioration of the retina and the loss of visual function. Therefore, lowering IOP can be an objective for the treatment of glaucoma patients, in order to decrease the potential for, or severity of, glaucomatous retinopathy. Unfortunately, many individuals do not respond well when treated with existing glaucoma therapies.

This invention is primarily concerned with the class of drugs, which exhibit HMG-CoA reductase inhibitory activity. The term "statin" has often been used to describe an "HMG-CoA reductase inhibitor." Statins have been used or evaluated in various medical studies for a variety of purposes. A few of these studies are set forth below. However, with all of these studies, there has been no recognition that statins are capable of being effective in the treatment of glaucoma and/or controlling or lowering intraocular pressure. The statins are a class of 3-hydroxy-3-methylglutaryl coenzyme A (HMG-CoA) reductase inhibitors, which are known for their clinical hypolipidemic properties. The hypolipidemic properties are due to the statins' ability to inhibit HMG-CoA reductase, preventing the conversion of 3-hydroxy-3-methylglutaryl-CoA to mevalonate, which is the rate-limiting step in cholesterol synthesis. However, statins also exert actions that may not be related to the inhibition of cholesterol biosynthesis. For example, statins have also been reported to inhibit the expression of various integrins, to inhibit superoxide anion production, to interfere with Rho protein isoprenylation, and to act as free radical scavengers, among other effects (Wassmann and Nickenig, Endothelium, 2003,10:23-33; Mason JC, Clin Sci (Lond) 2003,105:251-66). Also, the anti-inflammatory and cardiac protective activities of statins has been reported (Yoshida M J. Atheroscelrosis Thromb 2003, 10(3), 140-4; Luo JD and Chen AF Curr Med Chem 2003,10(16), 1593-601.

Additional examples of studies where statins have been used for non-glaucoma related purposes are further described in U.S. Patent Application No. 2003/0065020 which relates to the use of HMG-CoA reductase inhibitors, statins, to treat macular degeneration or prevent macular degeneration. Also, U.S. Patent No. 6,569,461 relates to the use of a delayed release formulation of certain dihydroxy open acid statins for the treatment of diabetic retinopathy. Schmidt et al. (Ophthalmic Res. 1994,26, 352-60) reported that patients treated with lovastatin or simvastatin, administered orally for at least one year, had no clinically significant change in intraocular pressure.

Clofibrate, a member of the fibrate class of peroxisome proliferator activated receptor (PPAR) agonist that produces a moderate reduction in LDL cholesterol and triglycerides, has also been reported to lower IOP.

WO 2005/053683, EP-A-0473337 and US-A-5134124 describe the use of statins in the treatment of glaucoma.

The present inventors, through significant studies, have discovered methods to treat glaucoma and/or control or lower intraocular pressure with the use of HMG-CoA reductase inhibitors, such as statins.

### SUMMARY OF THE PRESENT INVENTION

The present invention provides uses of statins selected from rosuvastatin, pitavastatin, berivastatin, glenvastatin, or a derivative thereof, or a combination thereof, in the manufacture of a medicament for the treatment of glaucoma in a patient, for controlling normal or elevated intraocular pressure in a patient, for preserving the trabecular meshwork of a patient, for protecting against ocular neurodegeneration in a patient, and for protecting against glaucomatous retinopathy in a patient, in accordance with claims which follow.

Additional features and advantages of the present invention will be set forth in part in the description that follows, and in part will be apparent from the description, or may be learned by practice of the present invention. The objectives and other advantages of the present invention will be realized and attained by means of the elements and combinations particularly pointed out in the description and appended claims.

To achieve these and other advantages and in accordance with the purposes of the present invention, as embodied and broadly described herein, the present invention relates to the use of compositions containing at least one HMG-CoA reductase inhibitor for the treatment of glaucoma and/or the lowering or controlling of normal or elevated IOP, such as IOP associated with normal-tension glaucoma and/or ocular hypertension. The compositions can be preferably pharmaceutical compositions, for instance, suitable for topical delivery to the eye.

It is to be understood that both the foregoing general description and the following detailed description are exemplary and exemplary only and are intended to provide a further explanation of the present invention, as claimed.

### BRIEF DESCRIPTION OF THE DRAWINGS

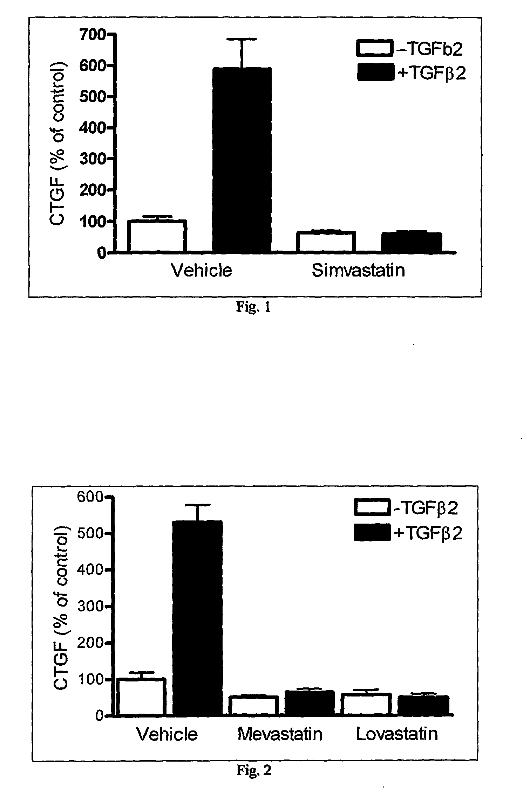

Figures 1-3 are graphs setting forth the effects of statins on basal andTGFp2-induced CTGF gene expression in cultured human trabecular meshwork cells.

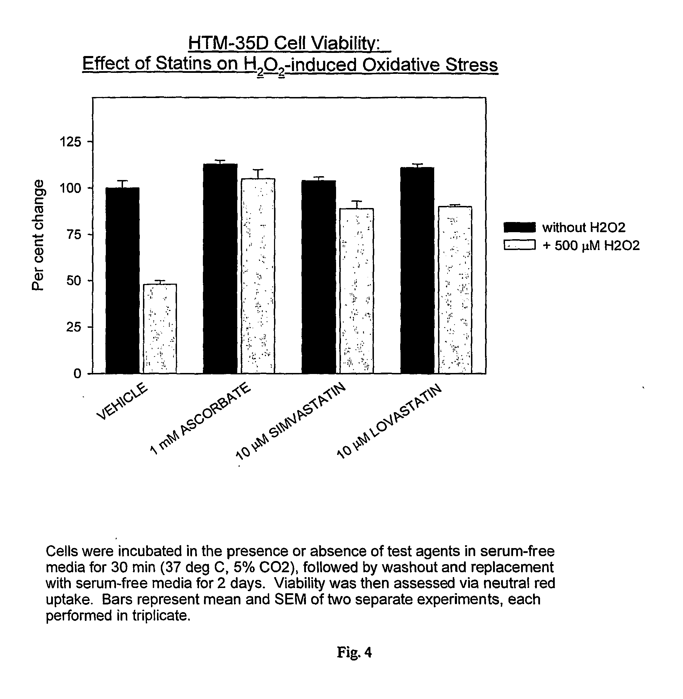

Figure 4 is a bar graph showing the effect of H202 induced oxidative stress in HTM-35D cell viability. Cells were incubated in the presence or absence of test agents in serum-free media for 30 min (37 deg C, 5% C02), followed by washout and replacement with serum-free media for 2 days. Viability was then assessed via neutral red uptake. Bars represent mean and SEM of two separate experiments, each performed in triplicate.

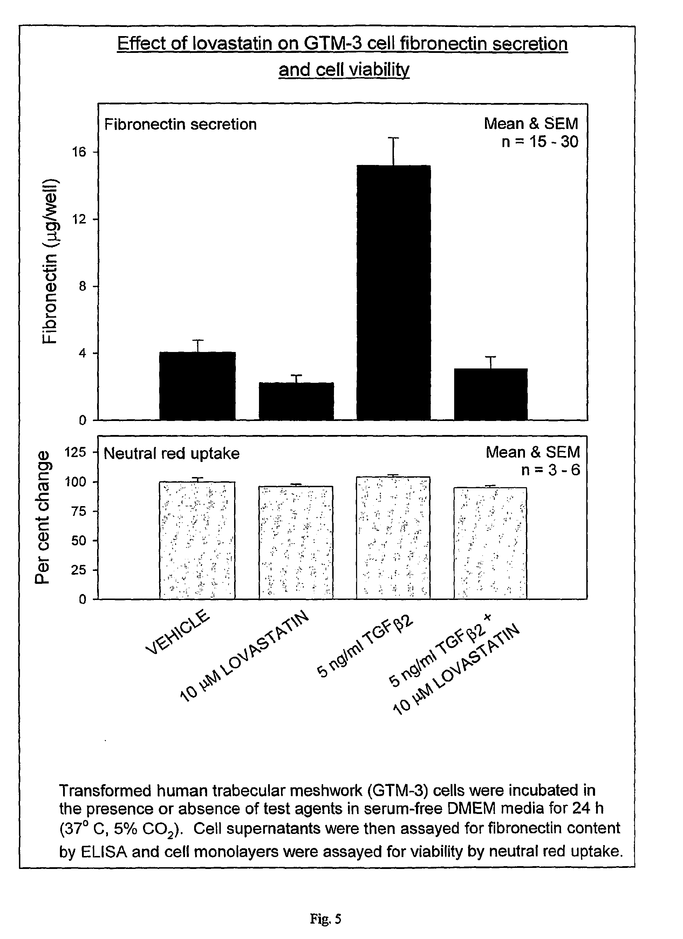

Figure 5 depicts bar graphs showing the effect of lovastatin on GTM-3 cell fibronectin secretion and viability. Transformed human trabecular meshwork (GTM-3) cells were incubated in the presence or absence of test agents inserum-free DMEM media for 24 h (37 C, 5% C02). Cell supernatants were then assayed for fibronectin content by ELISA and cell monolayers were assayed for viability by neutral red uptake.

### DETAILED DESCRIPTION OF THE PRESENT INVENTION

The present description in part relates to a compound for use in treating glaucoma in a human patient or other mammals. The present description also relates to a use to lower or control normal or elevated IOP in a human patient or other mammals. The Uses involve administering a composition containing at least one 3-hydroxy-3-methylglutaryl coenzyme A (HMG-CoA) reductase inhibitor. In other words, the present description relates to the use of at least one substance which has HMG-CoA reductase inhibitory activity.

One group of HMG-CoA reductase inhibitors which are suitable for use in the present invention is known as statins. For purposes of the present invention, "statins" as used herein are HMG-CoA inhibitors. These drugs can have hypolipidemic properties due to their ability to inhibit HMG-CoA reductase, preventing the conversion of 3-hydroxy-3methylglutaryl-CoA to mevalonate, which is the rate-limiting step in cholesterol synthesis.

Examples of HMG-CoA reductase inhibitors include rosuvastatin, pitavastatin, berivastatin, glenvastatin.

The chemical names are as follows:
rosuvastatin - ((3R,5S,6E)-7-[4-(4-fluorophenyl)-6-(1-methylethyl)-2-[methyl(methylsulfonyl)amino]-5-pyrimidinyl]-3,5-dihydroxy-6-heptenoic acid),
pitavastatin - ((3R,5S,6E)-7-[2-cyclopropyl-4-(4-fluorophenyl)-3-quinolinyl]-3,5-dihydroxy-6-heptenoic acid),
berivastatin - ((R*, S*-(E)-7-(4-(4-fluorophenyl)spiro(2H-1-benzopyran-2,1'-cyclopentan)-3-yl)-3,5-dihydroxy-ethyl ester),
glenvastatin - ((4R,65)-6-[(1E)-2-[4-(4-fluorophenyl)-2-(1-methylethyl)-6-phenyl-3 -pyridinyl]ethenyl]tetrahydro-4-hydroxy-2H-Pyran-2-one). 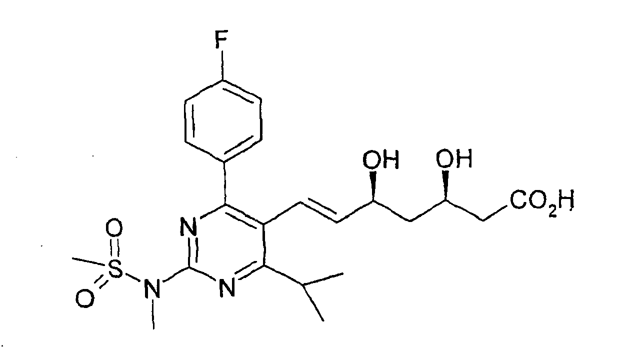
Rosuvastatin - (3R,5S,6E)-7-[4-(4-fluorophenyl)-6-(1 -methylethyl)-2-[methyl(methylsulfonyl)amino]-5- pyrimidinyl]-3,5-dihydroxy-6-heptenoic acid.
It is recognized that the above-described compounds can contain one or more chiral centers. This invention contemplates all enantiomers, diastereomers, and mixtures thereof.

The HMG-CoA reductase inhibitors utilized in the present invention also include compounds that have or include the above-described Structure B moiety in combination with moieties other than the Structure A moieties shown above.

The pharmaceutically acceptable salts and solvates, and prodrug forms of the above-described compounds can also be used in the methods of the present invention. Furthermore, derivatives of the compounds set forth above can also be used in the methods of the present invention. Derivatives include: derivatives of carboxylic acids (for example: carboxylic acid salts, esters, lactones, amides, hydroxamic acids, alcohols, esterified alcohols and alkylated alcohols (alkoxides)) and derivatives of alcohols (for example: esters, carbamates, lactones, carbonates, alkoxides, acetals, ketals, phosphates, and phosphate esters). With respect to the above-described compounds, several compounds contain fluorine on one or more of the aromatic rings, aud thus instead of a fluorine, any other halide can be used. Also, in lieu of hydrogen or alkyl groups as set forth in the compounds above, different alkyl groups can be used. For instance, instead of an -OH group, an -O-alkyl group could be used. Thus, various derivatives can easily be used in the present invention based on the guidance and knowledge presented herein, together with the knowledge that one skilled in the art has in this technical area.

The above examples are either commercially available (e.g., Calbiochem-Novabiochem Corporation, SST Corporation, Aceto Corporation, Tocris Cookson Inc., Sigma, and BIOMOL Research Laboratories, Inc.) or can be synthesized based on literature available to those skilled in the art.

The oral bioavailability of the statins is low (i.e., generally ranging from 5 to 20%) due to extensive pre-systemic elimination in the liver and intestinal mucosa. In addition, most statins are highly bound to plasma and are rapidly eliminated (Klotz U Arzneim Forsch (Drug Research) 2003, 53, 605-611). These factors significantly limit the amount of active compound that reaches ocular tissues following oral administration.

The use of topical ocular administration is therefore strongly preferred in the present invention. However, the present inventors have found that while certain statins are able to reach intraocular tissues via penetration of the cornea following topical ocular administration (e.g., pravastatin), other statins do not readily penetrate the cornea and consequently are not viable therapeutic agents in the methods of the present invention via this route of administration (e.g., atorvastatin).

The use of reverse phase high pressure liquid chromatography retention parameters such as retention index ("RI") have proven useful in predicting the partition of molecules into and through biological membranes. (Brent DA. Sabaltks JJ, Minick DJ, and Henry DW. Journal of Medicinal Chemistry 1983, 26, 1014-1020). The present inventors have discovered that RI values can be utilized to predict the ability of statins to reach therapeutic concentrations in intraocular tissues following topical ocular administration. Specifically, the inventors have found that statins having an RI value of 0.2 to 0.7 can be administered to patients via topical ocular administration. The RI values are determined by means of the procedure described below.

### Determination of Retention Index ("RI") Values

The test article is dissolved in solvent to provide a 15-20 µg/mL solution. This solution and a reference solution (PGF_{2α}isopropyl ester 1% in ethanol diluted in 3 mL diluent solvent) are injected directly into a Microsorb-MV ODS reverse phase high pressure chromatography column (4.6 mm ID X 15 cm length, 5 µm). The mobile phase is an aqueous pH 3.0 ammonium phosphate buffer / actonitrile (1:1) mixture with a flow rate of about 1 mL per minute. The retention index (RI) was calculated using the following formula:

RI = RT1/RT2

wherein:
RT1 = retention time of the test article, and
RT2 = retention time of PGF_{2α}isopropyl ester standard.

The above-described RI value range of 0.2 to 0.7 is applicable to all of the statins described herein. However, the range is based on the dihydroxy open acid form of the compounds. Consequently, the statins wherein Structure B is in the lactone configuration must be converted to the corresponding open acid before determining the RI value.

The compounds of the present invention can be used to lower and control IOP including IOP associated with normotension glaucoma, ocular hypertension, and glaucoma in warm blooded animals, particularly humans. The compositions are preferably formulated pharmaceutical compositions which are preferably suitable for topical delivery, such as to the eye of the patient. The composition can be administered in any suitable fashion by conventional techniques.

Statins can help delay/counteract the loss of trabecular meshwork (TM) cellularity seen with glaucoma. Even though the exact mechanism is not known, the anti-oxidative and free-radical scavenging effects of statins can counteract age- and/or glaucoma-related oxidative damage/stress in the TM. A decrease in cellularity has been noted previously in TM tissue from glaucoma patients (Alvarado et al, Ophthalmology, 1984, 91:564-579). Furthermore, it has been reported that lipid peroxidation product levels were increased at least two-fold in aqueous humor from glaucoma patients, along with a decrease in the total antioxidative capacity (Kurysheva et al, Vestn Oftalmol, 1996, 112:3-5). Increased levels of free radicals can be damaging to a wide variety of tissues, and therefore statins can help delay/counteract the loss of TM cellularity seen with glaucoma. The TM is the major site of aqueous humor (AH) drainage and compromised AH outflow through the TM can be a causative factor for the increased IOP often noted in glaucoma. By preserving TM cellularity by treatment with statins, AH outflow can be improved and IOP can be decreased. Thus, the present invention further relates to a method to preserve the TM of a patient by administering a pharmaceutically effective amount of a composition containing at least one HMG-CoA reductase inhibitor to a patient.

The present invention can also prevent the accumulation of excess or inappropriate extracellular matrix that can occlude the outflow pathway. An over accumulation of extracellular matrix materials in the region of the TM is also a hallmark of many forms of glaucoma. Such increases can lead to increased resistance to aqueous outflow, thereby elevating IOP. Connective tissue growth factor (CTGF) is implicated as a causative factor in conditions associated with the over accumulation of ECM, e.g., sclerodoma, fibroproliferative diseases, and scarring. CTGF can increase the production of extracellular matrix (ECM) materials such as collagen I and fibronectin. The presence of CTGF gene products has been detected in both isolated tissues and cell lines established from human TM, and the ECM component fibronectin is actively-secreted by cultured human TM cells. Statin drugs such as simvastatin, lovastatin, and mevastatin (compactin) significantly reduce connective tissue growth factor (CTGF) gene expression by cultured human TM cells. Statins also significantly reduce fibronectin secretion by cultured human TM cells. Therefore, via anti-oxidative and free radical-scavenging activities, as well via suppression of CTGF expression and fibronectin secretion, statin agents represent a novel potential means to lower IOP.

Complementing the ocular hypotensive effect, statins can exert a neuroprotective effect. Elevated levels of mediators of neurodegeneration, such as iNOS, TNFα, matrix metalloproteinases (MMP) and activated astrocytes, have been observed in the glaucomatous retina.

One means of neuroprotection may be due to a reduction of ischemic damage to neuronal tissue. Statins upregulate endothelial nitric oxide synthase, and thus may help preserve blood flow to ischemic tissues. Statins also have been shown to protect retinal neurons in a model of ischemia-reperfusion injury, an effect which was blocked by an inhibitor of nitric oxide synthase.

Furthermore, the anti-oxidative and free-radical scavenging effects of statins can be protective against oxidative damage/stress in the retina and optic nerve related to glaucoma. Glaucoma causes death of the retinal ganglion cell (RGC). The exact molecular mechanism(s) is unclear. However, there are three popular hypotheses: (1) retinal vascular abnormality leading to ischemia (Flammer et al, Prog Retin Eye Res, 2002, 21:359-393), (2) glutamate toxicity (Dreyer et al, Arch Ophthalmol, 1996, 114:299-305), and (3) withdrawal of neurotrophic factors (Quigley et al, Arch Ophthalmol, 1981, 99:635-649; Quigley et al, Arch Ophthalmol, 1982, 100:135-146; Quigley et al, Am J Ophthalmol, 1983, 95:674-691). All of these proposed mechanisms can lead to an oxidative toxicity to the RGC in glaucoma and contribute to its death. For example, excessive amounts of free radicals and reactive oxygen species can be generated by ischemia, which depletes ATP production, causes mitochondrial dysfunction and increases free radicals. Glutamate toxicity increases intracellular calcium concentration, activates calpains and NO synthase and produces free radicals. Withdrawal of neurotrophic factors has also been shown to cause oxidative damage (Greenlund et al, Neuron, 1995, 14:303-315). Based on this information, antioxidants, such as the statins, are useful as neuroprotectants in glaucoma patients. The present invention further relates to a method to protect against glaucomatous retinopathy of a patient by administering a pharmaceutically effective amount of a composition containing at least one HMG-CoA reductase inhibitor to the patient.

The compositions used in the present invention can be various types of pharmaceutical compositions, such as ophthalmic formulations for delivery to the eye (e.g., topically, intracamerally, or via an implant). The compositions are preferably topical ophthalmic formulations for delivery to the eye. The compositions may include ophthalmologically acceptable preservatives, viscosity enhancers, penetration enhancers, buffers, sodium chloride, and water to form an aqueous, sterile ophthalmic suspension or solution. Ophthalmic solution formulations may be prepared by dissolving the active ingredient in a physiologically acceptable isotonic aqueous buffer. Further, the ophthalmic solution may include an ophthalmologically acceptable surfactant to assist in dissolving the active ingredient. Furthermore, the ophthalmic solution may contain an agent to increase viscosity, such as hydroxymethylcellulose, hydroxyethylcellulose, hydroxypropylmethylcellulose, methylcellulose, polyvinylpyrrolidone, or the like, to improve the retention of the formulation in the conjunctival sac. Gelling agents can also be used, including, but not limited to, gellan and xanthan gum. In order to prepare sterile ophthalmic ointment formulations, the active ingredient is combined with a preservative in an appropriate vehicle, such as, mineral oil, liquid lanolin, or white petrolatum. Sterile ophthalmic gel formulations may be prepared by suspending the active ingredient in a hydrophilic base prepared from the combination of, for example, carbopol-974, or the like, according to the published formulations for analogous ophthalmic preparations; preservatives and tonicity agents can be incorporated.

The compositions used in the present invention are preferably formulated as topical ophthalmic suspensions or solutions, with a pH for instance of from about 4 to about 8. The establishment of a specific dosage regimen for each individual is left to the discretion of the clinicians: The active ingredient (e.g., statin) will normally be contained in these formulations in an amount from about 0.01 % to about 10% by weight of the composition or formulation, such as from about 0.01% to about 5% by weight, and preferably in an amount from about 0.05% to about 2% and most preferably in an amount of from about 0.1 to about 1.0% by weight. The dosage form may be a solution or suspension microemulsion. For instance, for topical application, 1 to 2 drops of these formulations would be delivered to the surface of the eye from about 1 to about 4 times per day according to the discretion of a skilled clinician. The compositions of the present invention can be administered as a single dose or as multiple doses. The compositions of the present invention can be administered for one day or multiple days and the composition of the present invention can be administered for consecutive days or can be administered on a schedule where a treatment occurs on one day but not on the next day. In other words, the treatment can occur on non-consecutive days. The treatment can occur for at least two consecutive days, at least three consecutive days, or more. Furthermore, multiple dosages can occur on the same day, over consecutive days, or over non-consecutive days. Any combination of dose regimen is possible.

The compositions can also be used in combination with other agents for treating glaucoma, such as, but not limited to, β-blockers (e.g., timolol, betaxolol, levobetaxolol, carteolol, levobunolol, propranolol), carbonic anhydrase inhibitors (e.g., brinzolamide and dorzolamide), α1 antagonists (e.g., nipradolol), α2 agonists (e.g. iopidine and brimonidine), miotics (e.g., pilocarpine and epinephrine), prostaglandin analogs (e.g., latanoprost, travoprost, bimatoprost, unoprostone, and compounds set forth in U.S. Patent Nos. 5,889,052; 5,296,504; 5,422,368; 5,352,708; and 5,151,444), and neuroprotectants (e.g., compounds from U.S. Patent No. 4,690,931, particularly eliprodil and R-eliprodil, as set forth in application U.S.S.N. 60/203,350), and appropriate compounds from WO 94/13275, including memantine.

### EXAMPLE 1

HMG-CoA Reductase Inhibition Assay

HMG-CoA reductase activity can be assessed by the following procedure of Shefer et al. [J. Lipid Res 1972, 13, 402] as described in U.S. Published Patent Application No. US 2003/0065020. The complete assay medium contained the following in a total volume of 0.8 mL; phosphate buffer, pH7.2, 100mM; MgCl₂ , 3mM; NADP, 3mM; glucose-6-phosphate dehydrogenase, 3 enzyme units; reduced glutathione 50mM; HMG-CoA (glutaryl-3-¹⁴C), 0.2 mM (0.1 µCi); and partially purified enzyme stock solution, 100 µL.

Test compounds in the acid salt form were added to the assay system in 10-µL volumes at selected concentrations. After a 40-minute incubation at 37°C with shaking and exposure to air, the reaction was stopped by the addition of 0.4 mL of 8 N HCl. After an additional 30-minute incubation at 37°C to ensure the complete lactonization of mevalonic acid to mevalonolactone, 0.2 mL of the mixture was added to an 0.5 x 0.5 cm column containing 100-200 mesh Bio-Rex 5, chloride form (Bio-Rad), wetted with distilled water as described by Alberts et al. [Proc Natl. Acad. Sci. U.S.A. 1980, 77, 3967]. The unreacted (C¹⁴]HMG-CoA was absorbed on the resin and the [C¹⁴]mevalonate was eluted with distilled water (2 x 1mL) directly into 7-mL scintillation vials. Five milliliters of Aquasol-2 were added to each vial, and radioactivity measured in a scintillation counter. IC₅₀ values were determined by plotting percentage inhibition against test compound concentration and fitting a straight line to the resulting data by using the least-squares methods. For estimation of relative inhibitory potencies, compactin was used as a standard and was assigned a value of 100 and the IC₅₀ value of the test compound was compared with that of compactin determined simultaneously.

### Reference EXAMPLE 2

Inhibition of TGFβ2 stimulated CTGF gene expression

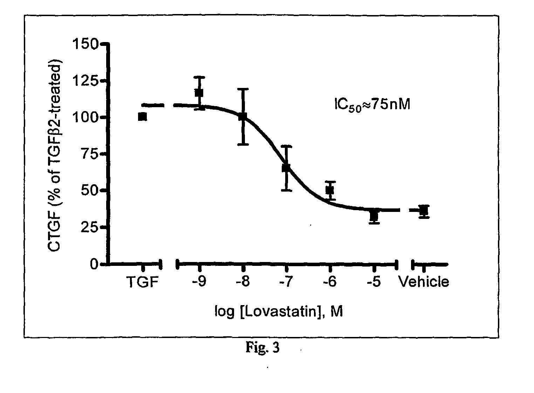

The effectiveness of statins on CTGF gene expression in cultured human trabecular meshwork (TM) cells was studied. The results are summarized in Figures 1-3. In these experiments, the CTGF/18S cDNA levels were measured by quantitative reverse-transcription PCR (QPCR) and compared. As can be seen from the summary of the results in Figures 1 and 2, three different types of statins were tested to determine the effect on controlling CTGF levels. As can be seen from the Figures, when TGFβ2 was present in the vehicle, the CTGF levels were quite high. However, when one of the statins was introduced, these levels were significantly lowered on the order of over 100%. Lovastatin inhibited TGFβ2-stimulated CTGF expression in a dose-dependent manner with an IC₅₀ value of 75nM (Fig. 3). The results of this study clearly demonstrate that statins have a great effect on the CTGF gene expression in cultured human trabecular meshwork cells.

### Reference EXAMPLE 3

Protection against H₂O₂ mediated TM cell death

The effect of H₂O₂-induced oxidative stress on HTM-35 D cell (a cultured human TM cell strain) viability was tested with and without a statin present. In particular, cells were incubated in the presence or absence of test agents in serum-free media for 30 minutes (37°C, 5% CO2), followed by the washout and replacement with serum-free media for two days. The viability was then assessed via neutral red uptake. The results are summarized in Figure 4, wherein the bars represent the mean and SEM of two separate experiments, each performed in triplicate. As can be seen in Figure 4, with the presence of a statin, the effects of H₂O₂ on HTM-35D cell viability were suppressed or controlled to essentially levels comparable to where no H₂O₂ was present. Thus, these tests show the ability of the statins to be useful in the treatment of glaucoma and to control or lower IOP.

### Reference EXAMPLE 4

Inhibition of TM fibronectin secretion

The effect of lovastatin on both basal (vehicle) and stimulated (TGFβ2) GTM-3 (a transformed human TM cell strain) secretion of fibronectin was tested. GTM-3 cells were incubated in the presence or absence of test agents in serum-free media for 24 h (37° C, 5% CO₂), then changes in secreted fibronectin levels were determined by ELISA of cell supernatants. Neutral red uptake assays were performed on the remaining cell monolayers, in order to determine the effect of test agents on GTM-3 cell viability. The results are summarized in Figure 5. As can be readily seen in Figure 5, lovastatin suppressed TGFβ2-stimulated fibronectin secretion, in addition to suppression of basal secretion, without affecting cell viability. Thus, by virtue of the ability to reduce the excessive accumulation of extracellular matrix (e.g. fibronectin), statins are useful in the treatment of glaucoma and to control or lower IOP.

### Reference EXAMPLE 5

Acute IOP Response in Lasered (Hypertensive) Eyes of Conscious Cynomolgus Monkeys.

Intraocular pressure (IOP) was determined with an Alcon Pneumatonometer after light corneal anesthesia with 0.1% proparacaine. Eyes were washed with saline after each measurement. After a baseline IOP measurement, the test compound was instilled in one 30 µL aliquot to the right eyes only of nine cynomolgus monkeys. The vehicle was instilled in the right eyes of six additional animals. Subsequent IOP measurements were taken at 1, 3, and 6 hours. Lovastatin, fluvastatin, atorvastatin and pravastatin did not significantly lower IOP following a single topical ocular application of 300 µg.

### Reference EXAMPLE 6

Response in Lasered (Hypertensive) Eyes of Conscious Cynomolgus Monkeys following repeated dosing.

In a further study, the intraocular pressure-lowering efficacy of lovastatin was measured in conscious ocular hypertensive monkeys using a five-dose, three-day study design. The test item was administered in one 30 µL aliquot to the lasered (unilateral laser trabeculoplasty) eye of eight animals. The vehicle was instilled in the lasered eyes of six additional animals as a control. Doses 1, 3, and 5 were instilled at 0900 hours on three consecutive days; doses 2 and 4 at 1630 hours on days 1 and 2. IOP was measured at 1, 3 and 6 hours after doses 1 and 5. IOP was also measured at 16 hours after dose 4. The percent change from time 0 baseline was determined for each animal for every IOP measurement.

Lovastatin, lowered IOP in the lasered monkey eye by an average of 22.8% (8.3 mmHg), 21.5% (8.1 mmHg) and 25.5% (9.5 mmHg) at 1, 3, and 6 hours, respectively, in lasered monkeys after the fifth topical ocular instillation of 300 µg. There was an average reduction in IOP of 12.5% in the control group. In a subsequent study, wherein nine doses of lovastatin were administered over a five day period, IOP was reduced by up to 7.4%.

The following formulations can be made for purposes of the present invention.

### Reference EXAMPLE 7

| **Ingredients** | **Amount (wt. %)** |
|---|---|
| Statin, such as lovastatin | 0.01 - 2% |
| Hydroxypropyl methylcellulose | 0.5% |
| Dibasic sodium phosphate (anhydrous) | 0.2% |
| Sodium chloride | 0.5% |
| Disodium EDTA (Edetate disodium) | 0.01 % |
| Polysorbate 80 | 0.05% |
| Benzalkonium chloride | 0.01% |
| Sodium hydroxide / Hydrochloric acid | For adjusting pH to 7.3 - 7.4 |
| Purified water | q.s. to 100% |

| **Ingredients** | **Amount (wt. %)** |
|---|---|
| Statin, such as lovastatin | 0.01 - 2% |
| Methyl cellulose | 4.0% |
| Dibasic sodium phosphate (anhydrous) | 0.2% |
| Sodium chloride | 0.5% |
| Disodium EDTA (Edetate disodium) | 0.01 % |
| Polysorbate 80 | 0.05% |
| Benzalkonium chloride | 0.01% |
| Sodium hydroxide / Hydrochloric acid | For adjusting pH to 7.3 - 7.4 |
| Purified water | q.s. to 100% |

| **Ingredients** | **Amount (wt. %)** |
|---|---|
| Statin, such as lovastatin | 0.01 - 2% |
| Guar gum | 0.4- 6.0% |
| Dibasic sodium phosphate (anhydrous) | 0.2% |
| Sodium chloride | 0.5% |
| Disodium EDTA (Edetate disodium) | 0.01% |
| Polysorbate 80 | 0.05% |
| Benzalkonium chloride | 0.01 % |
| Sodium hydroxide / Hydrochloric acid | For adjusting pH to 7.3 - 7.4 |
| Purified water | q.s. to 100% |

| **Ingredients** | **Amount (wt. %)** |
|---|---|
| Statin, such as lovastatin | 0.01 - 2% |
| White petrolatum and mineral oil and lanolin | Ointment consistency |
| Dibasic sodium phosphate (anhydrous) | 0.2% |
| Sodium chloride | 0.5% |
| Disodium EDTA (Edetate disodium) | 0.01% |
| Polysorbate 80 | 0.05% |
| Benzalkonium chloride | 0.01% |
| Sodium hydroxide / Hydrochloric acid | For adjusting pH to 7.3 - 7.4 |

## Claims

1. Use of a statin selected from rosuvastatin, pitavastatin, berivastatin, glenvastatin, or a derivative thereof, or a combination thereof, in the manufacture of a medicament for the treatment of glaucoma in a patient.

2. Use of a statin selected from rosuvastatin, pitavastatin, berivastatin, glenvastatin, or a derivative thereof, or a combination thereof, in the manufacture of a medicament for controlling normal or elevated intraocular pressure in a patient.

3. Use of a statin selected from rosuvastatin, pitavastatin, berivastatin, glenvastatin, or a derivative thereof, or a combination thereof, in the manufacture of a medicament for preserving the trabecular meshwork of a patient.

4. Use of a statin selected from rosuvastatin, pitavastatin, berivastatin, glenvastatin, or a derivative thereof, or a combination thereof, in the manufacture of a medicament for protecting against ocular neurodegeneration in a patient.

5. Use of a statin selected from rosuvastatin, pitavastatin, berivastatin, glenvastatin, or a derivative thereof, or a combination thereof, in the manufacture of a medicament for protecting against glaucomatous retinopathy in a patient.

6. Use as in any of claims 1 to 5, wherein said medicament is for topical administration to at least one eye of said patient.

7. Use as in claim 6, wherein said statin has a reverse phase high pressure liquid chromatography retention index (RI) value of 0.2 to 0.7.

8. Use as in any of claims 1 to 5, wherein said medicament is for intraocular administration to at least one eye of said patient.

9. A composition comprising a statin selected from rosuvastatin, pitavastatin, berivastatin, glenvastatin, or a derivative thereof, or a combination thereof, from 0.05% to 2.0% by weight of said composition, for use in the treatment of glaucoma in a patient, controlling normal or elevated intraocular pressure in a patient, for preserving the trabecular meshwork in a patient, for protecting against ocular neurodegeneration in a patient, or for protecting against glaucomatous retinopathy in a patient.

10. The composition of claim 9, further comprising, a β-blocker, a carbonic anhydrase inhibitor, an α1 antagonist, an α2 agonist, a miotic, a prostaglandin analog, a neuroprotectant, or any combination thereof.

11. The composition of claim 9, further comprising, at least one carbonic anhydrase inhibitor.

## Patentansprüche

1. Verwendung von Statin, welches aus Rosuvastatin, Pitavastatin, Berivastatin, Glenvastatin oder einem Derivat daraus oder einer Kombination daraus ausgewählt ist, bei der Herstellung von einem Medikament zur Behandlung von Glaukom bei einem Patienten.

2. Verwendung von Statin, welches aus Rosuvastatin, Pitavastatin, Berivastatin, Glenvastatin oder einem Derivat daraus oder einer Kombination daraus ausgewählt ist, bei der Herstellung von einem Medikament zur Steuerung eines normalen oder erhöhten Augeninnendrucks bei einem Patienten.

3. Verwendung von Statin, welches aus Rosuvastatin, Pitavastatin, Berivastatin, Glenvastatin oder einem Derivat daraus oder einer Kombination daraus ausgewählt ist, bei der Herstellung von einem Medikament zur Einhaltung des Trabekelwerks eines Patienten.

4. Verwendung von Statin, welches aus Rosuvastatin, Pitavastatin, Berivastatin, Glenvastatin oder einem Derivat daraus oder einer Kombination daraus ausgewählt ist, bei der Herstellung von einem Medikament zum Schutz gegen Augen-Neurodegeneration bei einem Patienten.

5. Verwendung von Statin, welches aus Rosuvastatin, Pitavastatin, Berivastatin, Glenvastatin oder einem Derivat daraus oder einer Kombination daraus ausgewählt ist, bei der Herstellung von einem Medikament zum Schutz gegen Glaukom-Retinopathie bei einem Patienten.

6. Verwendung nach einem der Ansprüche 1 bis 5, bei welcher das Medikament zur topischen Abgabe an zumindest ein Auge des Patienten dient.

7. Verwendung nach Anspruch 6, bei welcher das Statin einen Rückwärtsphase-Hochdruckflüssigkeit-Chromatographie-Retentionsindex(RI)-Wert von 0,2 bis 0,7 hat.

8. Verwendung nach einem der Ansprüche 1 bis 5, bei welcher das Medikament zur intraokularen Abgabe an zumindest ein Auge des Patienten dient.

9. Zusammensetzung, welche ein Statin enthält, welches aus Rosuvastatin, Pitavastatin, Berivastatin, Glenvastatin oder einem Derivat daraus oder einer Kombination daraus von 0,05 % bis 2,0 % Gewichtseinheit der Zusammensetzung ausgewählt ist, zur Verwendung bei der Behandlung von Glaukom bei einem Patienten, zur Steuerung eines normalen oder erhöhten Augeninnendruckes bei einem Patienten, zur Einhaltung des Trabekelwerkes bei einem Patienten, zum Schutz gegen Augen-Neurodegeneration bei einem Patienten oder zum Schutz gegen Glaukom-Retinopathie bei einem Patienten.

10. Zusammensetzung nach Anspruch 9, welche ferner einen β-Blocker, einen Carboanhydrase-Hemmstoff, einen α1-Antagonisten, einen α2-Agonisten, eine Miotik, einen Prostaglandin-Analog, einen Neuroprotektanten oder jegliche Kombination daraus enthält.

11. Zusammensetzung nach Anspruch 9, welche zumindest einen Carboanhydrase-Hemmstoff enthält.

## Revendications

1. Utilisation d'une statine sélectionnée parmi la rosuvastatine, pitavastatine, bérivastatine, glenvastatine, ou un dérivé de ces dernières, ou une combinaison de ces dernières, dans la fabrication d'un médicament pour le traitement du glaucome chez un patient.

2. Utilisation d'une statine sélectionnée parmi la rosuvastatine, pitavastatine, bérivastatine, glenvastatine, ou un dérivé de ces dernières, ou une combinaison de ces dernières, dans la fabrication d'un médicament pour commander la pression intraoculaire normale ou élevée chez un patient.

3. Utilisation d'une statine sélectionnée parmi la rosuvastatine, pitavastatine, bérivastatine, glenvastatine, ou un dérivé de ces dernières, ou une combinaison de ces dernières, dans la fabrication d'un médicament pour préserver le trabéculum cornéoscléral d'un patient.

4. Utilisation d'une statine sélectionnée parmi la rosuvastatine, pitavastatine, bérivastatine, glenvastatine, ou un dérivé de ces dernières, ou une combinaison de ces dernières, dans la fabrication d'un médicament pour une protection contre une neurodégénérescence oculaire chez un patient.

5. Utilisation d'une statine sélectionnée parmi la rosuvastatine, pitavastatine, bérivastatine, glenvastatine, ou un dérivé de ces dernières, ou une combinaison de ces dernières, dans la fabrication d'un médicament pour une protection contre une rétinopathie glaucomateuse chez un patient.

6. Utilisation selon l'une quelconque des revendications 1 à 5, dans laquelle ledit médicament est destiné pour une administration topique à au moins un oeil dudit patient.

7. Utilisation selon la revendication 6, dans laquelle ladite statine a une valeur d'indice de rétention (RI) de chromatographie en phase liquide de pression élevée et en phase inverse de 0,2 à 0,7.

8. Utilisation selon l'une quelconque des revendications 1 à 5, dans laquelle ledit médicament est destiné pour une administration intraoculaire à au moins un oeil dudit patient.

9. Composition comportant une statine sélectionnée parmi la rosuvastatine, pitavastatine, bérivastatine, glenvastatine, ou un dérivé de ces dernières, ou une combinaison de ces dernières, à partir de 0,05 % à 2,0 % en poids de ladite composition, pour être utilisée dans le traitement du glaucome chez un patient, pour commander la pression intraoculaire normale ou élevée chez un patient, pour préserver le trabéculum cornéoscléral chez un patient, pour une protection contre une neurodégénérescence oculaire chez un patient, ou pour une protection contre une rétinopathie glaucomateuse chez un patient.

10. Composition selon la revendication 9, comportant de plus une β-bloquant, une inhibiteur d'anhydrase carbonique, un antagoniste α1, un agoniste α2, un miotique, un analogue de prostaglandine, un neuroprotecteur, ou toute combinaison de ces derniers.

11. Composition selon la revendication 9, comportant en outre, au moins un inhibiteur d'anhydrase carbonique.
